# FASCICULE DE BREVET EUROPEEN

(11) **EP 3 169 262 B1**
(45) Date de publication et mention de la délivrance du brevet: **27.11.2019**
(21) Numéro de dépôt: 15738647.5
(22) Date de dépôt: 16.07.2015
(51) Int. Cl.: A61B 18/20

(54) **ENSEMBLE DE TRAITEMENT À LUMIÈRE PULSÉE**
PULSLICHTTHERAPIEANORDNUNG
PULSED LIGHT THERAPY ASSEMBLY

(30) Priorité: 18.07.2014 FR 1456968
(43) Date de publication de la demande: 24.05.2017
(73) Titulaire: Eurofeedback, 91017 Evry Cedex (FR)
(72) Inventeur: SAFRAOUI, Georges, F-91140 Villejust (FR)
(74) Mandataire: Nony
(86) Numéro de dépôt international: PCT/EP2015/066343
(87) Numéro de publication internationale: WO 2016/009008

(56) Documents cités:
- WO-A2-2012/118862
- CN-A- 101 165 543
- FR-A1- 2 929 831
- US-A- 5 683 380
- US-A1- 2003 193 643
- US-A1- 2004 034 397
- US-A1- 2005 045 189
- US-A1- 2009 248 004
- US-A1- 2013 278 893
- US-B1- 6 612 697

## Description

La présente invention concerne les ensembles de traitement par lumière pulsée et les procédés de mise en œuvre correspondants.

Les machines LPP (Lumière Pulsée Polychromatique), encore appelées machines IPL (Intense Pulsed Light), sont largement utilisées aujourd'hui dans diverses applications tant cosmétiques que dermatologiques, et notamment pour l'épilation.

Elles se composent généralement d'un poste de base et d'une pièce à main qui est reliée au poste de base par un câble flexible. La pièce à main comporte une lampe flash qui génère des impulsions lumineuses de forte intensité. Le spectre de ces impulsions, avant filtration, s'étend de l'ultraviolet aux infrarouges, et la pièce à main comporte un filtre optique qui laisse passer essentiellement la lumière visible et le proche infrarouge, avec par exemple un pic vers 820 nm.

Dans le cas de l'utilisation de la machine, l'opérateur porte des lunettes qui atténuent l'intensité des flashs émis, tout en permettant de voir suffisamment pour pouvoir positionner la pièce à main précisément sur la région à traiter.

Les lunettes de protection existantes présentent des verres absorbants de couleur verte, plus ou moins foncée. Pour les verres les plus clairs, il est fréquent de voir l'opérateur prendre l'habitude de fermer les yeux juste avant d'appuyer sur la gâchette qui déclenche l'émission d'un flash, car même si le guide optique est appliqué fermement contre la peau, les fuites de lumière peuvent être sources d'inconfort visuel, voire de danger pour les opérateurs qui pratiquent à longueur de journée.

De manière surprenante, le marché des machines IPL s'est considérablement développé ces dernières années tout en conservant cet inconvénient d'utilisation.

Contrairement aux lasers dont l'utilisation est conditionnée par le port de lunettes respectant la norme EN 207, il n'existe actuellement aucune norme de protection occulaire dans l'utilisation de lumière pulsée IPL, dont la particularité est l'émission d'une lumière intense dans un spectre large allant du visible à l'infrarouge.

Les lunettes vertes de protection actuellement utilisées dans les machines IPL ne respectent que la norme EN 166 qui spécifie essentiellement la résistance mécanique des lunettes. Ces lunettes ne protègent pas la rétine de l'œil d'une façon satisfaisante.

Certaines machines offrent une possibilité de fonctionnement en rafale. Dans ce cas, l'action continue sur un poussoir ou une pédale permet à la pièce à main d'émettre une succession de flashs automatiques très rapprochés dans le temps, ce qui présente l'avantage de permettre à l'utilisateur d'effectuer un traitement en déplaçant de façon continue la pièce à main sur la zone à traiter. Il en résulte un gain de temps dans le traitement. Par contre, il n'est plus possible pour l'utilisateur, s'il le souhaite, de fermer les yeux durant le traitement, sauf à perdre en précision dans le geste. Il peut en résulter un inconfort visuel plus important, obligeant l'utilisateur à porter des lunettes aux verres plus sombres.

On a cherché à remédier à ce problème en proposant dans la demande US2010/0045882 A1 des lunettes à cristaux liquides dont l'obturation est commandée par une action exercée sur la gâchette de la pièce à main pour déclencher un flash. Les lunettes comportent un circuit de commande qui est informé qu'un flash va être déclenché et les cristaux liquides passent alors d'un état transparent à un état opaque, puis reprennent l'état transparent après l'émission du flash.

Un tel système est relativement complexe et coûteux, et d'un poids important. Il est en outre difficile d'éliminer tout risque de défaillance des lunettes. Or, l'émission des flashs doit absolument être empêchée si celles-ci ne passent pas dans l'état d'occultation. En outre, il est fait référence aux documents suivants: US2004/0034397 A1, WO2012/118862 A2 et US2003/0193643 A1.

L'invention vise à perfectionner encore les ensembles de traitement par lumière puisée de façon à permettre de traiter en toute sécurité les personnes, tout en offrant le meilleur confort visuel aux opérateurs, notamment en cas d'émission en rafale des flashs.

Elle y parvient grâce à un ensemble comportant :
- une machine de traitement notamment cosmétique ou dermatologique, par lumière pulsée comportant une pièce à main comportant une lampe flash et un premier filtre sélectif absorbant pour filtrer la lumière émise par la lampe flash avant sa sortie de la pièce à main,
- une paire de lunettes ou un masque de protection de l'opérateur, comportant un deuxième filtre sélectif absorbant pour filtrer la lumière incidente.

La transmission globale de la lumière visible et invisible (IR) à travers les premier et deuxième filtres est de préférence suffisamment faible pour que l'opérateur ne soit pas gêné visuellement par l'émission d'un flash ni agressé par une lumière infrarouge. Cette transmission globale est de préférence inférieure ou égale à 10 % sur la plage 580 nm à 1200 nm, mieux inférieure ou égale à 1 %, encore mieux inférieure ou égale à 0.1 % sur cette même plage. Cette plage peut correspondre sensiblement à la largeur du spectre de la lumière émise par la machine.

Par "agressé par une lumière infrarouge", il faut comprendre que le rayonnement infrarouge qui atteint l'utilisateur est supérieur aux normes de sécurité en vigueur, telles que définies dans la norme EN62471. Cela correspond à une énergie E(ir) < 18000.texp-0.75 (W/m²). Le niveau de protection recherché dans l'invention est de préférence bien plus important que la limite exigée par cette norme de sécurité. L'énergie maximum qui atteint la rétine est de préférence au minimum dix fois plus faible que la limite imposée par la norme.

Par « transmission globale » à la longueur d'onde λ, on désigne le produit des facteurs de transmission à la longueur d'onde λ de chacun des filtres. Par exemple, si le facteur de transmission à 650 nm du premier filtre est de 10⁻¹, et celui du deuxième à 650 nm est de 10⁻³, la transmission globale à 650 nm est de 10⁻⁴.

En dehors de la plage d'émission de la machine, et de préférence dans le spectre du visible, la transparence des lunettes de protection doit rester suffisamment élevée, notamment supérieure à 10%, et mieux supérieure à 95%, idéalement voisine de 100%, de façon à garder une bonne vision de l'environnement et de la zone à traiter. La transparence est définie comme le rapport entre l'intensité de la lumière qui sort du filtre et l'intensité de la lumière qui y entre. Le deuxième filtre absorbant a ainsi de préférence une transparence supérieure à 10%, mieux à 95% en dehors de la plage d'émission de la machine.

L'invention constitue une solution particulièrement élégante et économique pour améliorer le confort visuel des opérateurs et personnes traitées, et autorise l'émission de flashs en rafale tout en permettant de positionner précisément la pièce à main, grâce au fait que l'utilisateur peut garder un contrôle visuel total de son déplacement.

De préférence, l'invention permet d'obtenir un niveau de protection pour les machines IPL qui soit identique à celui exigé par la norme EN 207 pour les lasers, en particulier avec un facteur d'atténation de 10⁶, et notamment dans les infrarouges contre le risque thermique rétinien avec stimulus visuel faible (780 nm à 1 400 nm). Grace au niveau de protection offert par l'invention, il devient possible aux utilisateurs de fixer la zone de travail, pupilles dilatées, sans aucun danger.

De préférence, le filtre absorbant des lunettes ou du masque est de couleur bleue. Le filtre absorbant de la machine peut être agencé pour bloquer la lumière visible de longueur d'onde inférieure à λ_{c} nm, avec λ_{c} égal à 600 nm par exemple. Ce filtre absorbant peut être de couleur rouge.

On choisit de préférence les filtres absorbants des lunettes ou du masque d'une part, et celui de la pièce à main d'autre part, chacun avec une bande passante à front suffisamment raide. De préférence, le blocage de la lumiere de 10 à 90% du front s'effectue en moins de 100nm, ou mieux en moins de 50nm. Ceci permet d'éviter plus facilement un recouvrement des spectres. Les filtres à absorption offrent l'avantage d'avoir la même atténuation de lumière quel que soit l'angle d'incidence du rayon lumineux.

De préférence, la pièce à main comporte en amont du filtre absorbant un filtre dichroïque. Ce dernier peut avoir sensiblement le même spectre d'absorption que le premier filtre absorbant, par exemple la bande 400 nm à 650 nm. Ce filtre réduit le risque de dégradation du filtre absorbant.

De préférence, la machine est agencée pour émettre une rafale de flashs. Il s'agit par exemple de flashs émis à une fréquence de tirs d'au moins 1 Hz, pendant une durée qui va typiquement de 1 s à 1000s par exemple.

Le filtre absorbant des lunettes ou du masque a de préférence un facteur de transmission inférieur à 1 % pour λ < 400 nm et pour λ_{c} < λ < λ_{d}, avec λ_{c} égal à 600 nm par exemple et λ_{d} compris de préférence entre 850 et 1200 nm, notamment égal à 850 nm par exemple. On peut avoir pour λ compris entre λ_{c} et λ_{d} une transmission globale inférieure à 1%, mieux à 0,1%, encore mieux à 0,01%, avec λ_{c} inférieur à 850 nm et de préférence égal à 600 nm et λ_{d}> λ_{c} et de préférence égal à 850 nm.

Le deuxième filtre absorbant, c'est-à-dire celui des lunettes, a de préférence un facteur de transmission supérieur ou égal à 90 % pour au moins une longueur d'onde inférieure à λ_{c}, avec λ_{c} par exemple égal à 600 nm, et inférieur ou égal à 10 % pour les longueurs d'onde du domaine visible supérieures ou égales à 600 nm, soit dans la plage allant de 600 nm à 750 nm.

Le spectre d'émission de la machine de traitement est de préférence sensiblement nul pour λ ≤ 0.9.λ_{c}, *avec* λ_{c} égal à 580 ou 600 nm par exemple, notamment inférieur ou égal à 1 W/cm² et supérieur ou égal à 10 W/cm² pour 650 ≤ λ ≤ 800 nm, mieux 640 ≤ λ ≤ 830 nm, et notamment supérieur ou égal à 500 W/cm², mieux à 1000 W/cm², pour au moins une longueur d'onde dans l'intervalle 600 nm à 850 nm.

D'une façon générale, le spectre d'émission peut s'étendre jusqu'à 1200nm, et la puissance crête peut aller jusqu'à 2000W/cm². Dans ce cas, la longueur d'onde λ_{d} de fin de blocage de la lumière par les lunettes sera de 1200nm.

Le facteur de transmission des lunettes ou du masque peut être supérieur ou égal à 20% pour au moins une longueur d'onde du visible, de préférence dans la plage 400nm à λ_{c}, avec λ_{c} supérieur à 400 nm, et de préférence égal à 600 nm.

On peut avoir λ_{c} par exemple compris entre 530 nm et 710 nm, et par exemple égal à 530 nm, 610 nm ou 710 nm, dans des variantes de mise en œuvre de l'invention.

L'invention a encore pour objet, selon un autre de ses aspects, l'utilisation des lunettes ou du masque tels que définis plus haut, pour filtrer la lumière émise par la pièce à main.

L'invention pourra être mieux comprise à la lecture de la description qui va suivre, d'un exemple de mise en œuvre non limitatif de celle-ci, et à l'examen du dessin annexé, sur lequel :
- la figure 1 représente schématiquement un ensemble de traitement selon l'invention,
- la figure 2 représente schématiquement et partiellement la partie optique de la pièce à main,
- la figure 3 représente un masque de protection,
- la figure 4 est un exemple de spectre d'émission de la machine de traitement,
- la figure 5 représente un exemple de bande passante du filtre absorbant des lunettes dans des ensembles de traitement connus, et
- la figure 6 représente un exemple de bande passante du filtre absorbant des lunettes dans un ensemble selon l'invention, dont le spectre d'émission de la machine correspond à celui reproduit à la figure 4.

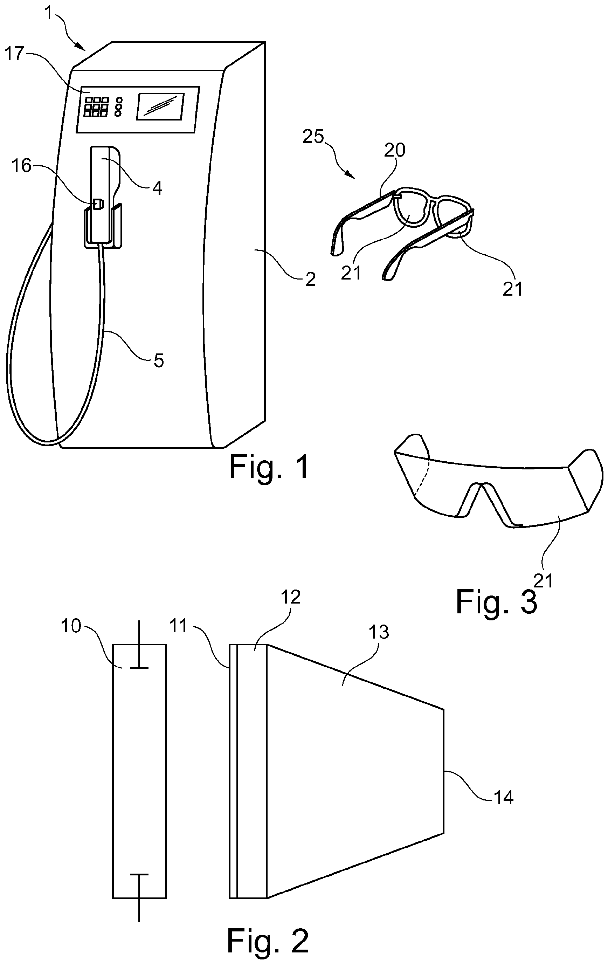

La machine 1 de traitement par lumière pulsée représentée à la figure 1 comporte un poste de base 2 qui contient un générateur électrique et une pièce à main 4 qui est reliée au poste de base par un câble flexible 5.

La pièce à main 4 peut être refroidie par une circulation d'eau à l'aide de conduites aller et retour intégrées au câble flexible 5.

La pièce à main 4 comporte comme illustré à la figure 2 une lampe flash 10 qui est reliée au générateur électrique, un filtre dichroïque 11 et un filtre absorbant 12 disposé en aval du filtre 11.

Les filtres 11 et 12 peuvent être placés en amont d'un conduit optique 1 3, qui guide la lumière vers une face de sortie 14 de la lumière, à appliquer sur la zone à traiter.

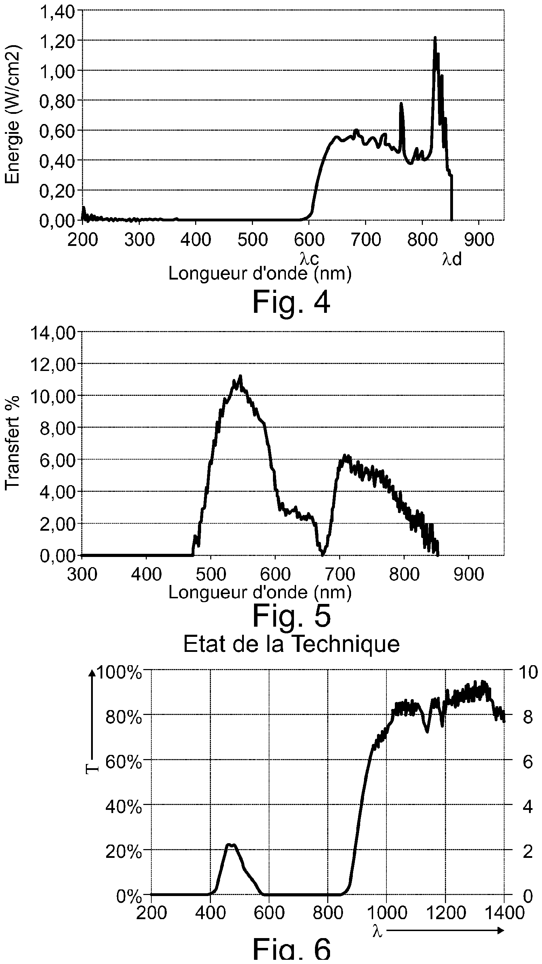

Le spectre énergétique de la lumière émise par la pièce à main a par exemple l'allure représentée à la figure 4.

La lampe flash 10 est refroidie par une circulation d'eau et les filtres dichroïque et absorbant sont de préférence réalisés sous une forme monolithique de façon à être refroidis par cette circulation d'eau.

Le filtre dichroïque 11 est avantageusement déposé sur le filtre absorbant 12, par une technique de dépôt sous vide, ce qui garantit un contact intime entre les deux et une grande efficacité de refroidissement du filtre absorbant 12 par l'eau.

Le filtre dichroïque 11 peut être réalisé avec le filtre absorbant 12 conformément à l'enseignement de la demande US2011/0071510 A1 au nom de la demanderesse.

Le conduit optique 13 est par exemple fixé de façon amovible sur le boîtier de la pièce à main 4 afin de permettre l'emploi de conduits optiques de différentes sections selon la nature du traitement.

La lampe flash 10 peut être contenue dans une cartouche reçue de façon amovible dans le boîtier de la pièce à main, afin de faciliter son remplacement, comme décrit par exemple dans le brevet EP 1 906 856 A1 de la demanderesse.

La pièce à main 4 comporte un interrupteur 16 commandé par l'utilisateur pour déclencher les flashs. Une pédale peut être utilisée en parallèle avec l'interrupteur 16.

Les caractéristiques des flashs, notamment l'énergie des flashs et leur fréquence d'émission, le cas échéant, peuvent être contrôlées à partir d'une interface utilisateur 17 prévue sur le poste de base.

Il est particulièrement avantageux que les flashs puissent être émis en rafale avec une fréquence relativement élevée.

Par exemple, les flashs sont émis avec une fréquence supérieure ou égale à 1Hz ou mieux 5Hz tant que l'utilisateur appuie sur l'interrupteur 16 de déclenchement des flashs. La fluence d'un flash est de préférence supérieure ou égale à 3J/cm² ou mieux supérieur à 6 J/cm² et elle est comprise par exemple entre 3 et 16 J/cm².

La lampe flash 10 est de préférence un tube à éclairs en quartz, rempli de xénon.

La bande passante du filtre absorbant 12 est de préférence choisie de façon à éliminer au mieux le rayonnement de longueur d'onde inférieure ou égale à λ_{c}; avec λ_{c} de préférence égal à 600 nm.

La bande passante du filtre dichroïque 11 est de préférence choisie pour éliminer le rayonnement de longueur d'onde inférieure ou égale à λ_{c} ainsi que le rayonnement infrarouge, notamment celui de longueur d'onde supérieure à 850 nm environ.

On choisit les caractéristiques du filtre 12 de façon à avoir de préférence pour sa bande passante un front relativement raide entre le domaine d'absorption pour les longueurs d'onde inférieures ou égales à λ_{c} et le domaine passant pour les longueurs d'ondes supérieures ou égales à λ_{c}.

Ce front peut se caractériser par une transition de 10 à 90% dans une bande spectrale inférieure à 100 nm, mieux inférieure à 50nm.

La machine de traitement 1 s'utilise avec des moyens de protection oculaire portés par l'opérateur qui manipule la pièce à main 4, constitués par exemple par des lunettes 25 comportant une monture 20 et des verres 21.

Ces derniers présentent, conformément à l'invention, des caractéristiques de filtration complémentaires de celles de la machine 1.

Ainsi, la lumière émise par la pièce à main est suffisamment filtrée par les lunettes 25 pour que la lumière résiduelle qui atteint les yeux de l'opérateur ne constitue pas une gêne, et que le rayonnement infrarouge ne constitue pas un danger.

On choisit la bande passante des lunettes 25 de façon à ce que l'absorption soit faible là où l'absorption du filtre 11, 12 est élevée, et élevée là où l'absorption du filtre 11, 12 est faible.

Ainsi, on a de préférence pour le filtre absorbant des lunettes 25 une transmission inférieure ou égale à 1 % dans la plage λ_{c} à λ_{d} où la machine 1 émet notablement, et une transmission supérieure ou égale à 10% pour au moins une longueur d'onde au sein d'une plage de longueurs d'onde inférieures à λ_{c}, avec λ_{c} de préférence égal à 600 nm et λ_{d} de préférence égal à 850 nm.

On a représenté à la figure 6 un exemple de bande passante de lunettes 25 adaptée au spectre d'émission représenté à la figure 4.

Dans une telle situation, les verres 21 des lunettes 25 apparaissent de couleur bleue et le filtre absorbant 12 de couleur rouge.

A titre de comparaison, on a représenté à la figure 5 le facteur de transmission de lunettes actuellement commercialisées avec les machines de traitement IPL, et qui posent les problèmes énoncés dans l'introduction.

Les lunettes 25 peuvent être sélectionnées parmi celles proposées par certains fabricants pour se protéger de la lumière émise par les lasers.

Dans l'exemple illustré, on peut ainsi choisir celles proposées par la société LASERVISION sous la référence commerciale FxxP1EO2

Bien entendu, lorsque le spectre d'émission diffère de celui illustré à la figure 4, la bande passante des lunettes 25 est modifiée en conséquence.

L'invention n'est pas limitée à l'exemple qui vient d'être décrit.

On peut par exemple remplacer les lunettes 25 par un masque de protection similaire à un masque de ski, par exemple tel qu'illustré à la figure 3.

La personne qui reçoit le traitement peut être équipée des mêmes lunettes 25.

Le filtre absorbant de la machine de traitement peut être constitué d'une pièce unique ou de plusieurs pièces disposées optiquement en série. Il en est de même du filtre absorbant des lunettes.

La valeur λ_{c} peut être autre que 600 nm, notamment être plus faible ou plus grande, par exemple égale à 510, 610 ou 710 nm, λ_{d} peut être autre que 850 nm, notamment être supérieure à 850 nm.

Le deuxième filtre absorbant, à savoir celui des lunettes, peut être réalisé monobloc en un seul matériau transparent traité dans la masse ou en variante au moyen de deux filtres accolés, ayant des bandes d'absorption spécifiques différentes. Par exemple, on accole un premier verre (organique ou minéral) filtrant les IR et un deuxième verre (organique ou minéral) filtrant les longueurs d'onde du domaine visible.

L'expression « comportant un » doit se comprendre comme étant synonyme de « comprenant au moins un », sauf si le contraire est spécifié.

## Revendications

1. Ensemble comportant :
- une machine de traitement par lumière pulsée (1) comportant une pièce à main (4) comportant une lampe flash (10) et un premier filtre absorbant (12) pour filtrer la lumière émise par la lampe flash avant sa sortie de la pièce à main,
- une paire de lunettes (25) ou un masque de protection de l'opérateur, comportant un deuxième filtre absorbant (21) pour filtrer la lumière incidente,
ensemble **caractérisé par le fait que** la transmission globale de la lumière visible et infrarouge à travers les premier et deuxième filtres est suffisamment faible dans la plage d'émission de la pièce à main pour que l'opérateur ne soit pas gêné visuellement par l'émission d'un flash, ni agressé par la lumière infrarouge, la transmission globale étant inférieure ou égale à 10 % sur la plage 580 nm à 1200 nm, le deuxième filtre absorbant ayant une transmission inférieure ou égale à 1% dans la plage de λ_{c} à λ_{d} d'émission de la machine, le facteur de transmission de la paire de lunettes (25) ou du masque de protection de l'opérateur étant supérieur ou égal à 20% dans une plage comprise entre 400 nm et λ_{c}, λ_{c} étant supérieur à 400 nm, le filtre absorbant des lunettes ou du masque ayant un facteur de transmission inférieur à 1% pour λ < 400 nm.

2. Ensemble selon la revendication 1, le deuxième filtre absorbant (21) ayant une transparence supérieure à 10%, mieux à 95% en dehors de la plage d'émission de la machine.

3. Ensemble selon la revendication 1 ou 2, la transmission globale dans la plage λ_{c} à λ_{d} étant inférieure à 1 %, mieux à 0.1 %, encore mieux à 0.01 %, avec λ_{c} inférieur à 850 nm et λ_{d} > λ_{c}.

4. Ensemble selon l'une quelconque des revendications 1 à 3, le filtre absorbant (21) des lunettes (25) ou du masque étant de couleur bleue.

5. Ensemble selon l'une quelconque des revendications 1 à 4, avec λ_{c} égal à 600 nm et λ_{d} compris entre 850 et 1200 nm.

6. Ensemble selon l'une quelconque des revendications précédentes, la pièce à main comportant en amont du filtre absorbant (12) un filtre dichroïque (11) qui filtre les infrarouges et réduit le risque de dégradation du filtre absorbant.

7. Ensemble selon l'une quelconque des revendications précédentes, la machine étant agencée pour émettre une rafale de flashs, notamment de flashs émis à une fréquence de tirs d'au moins 1 Hz, notamment pendant une durée qui va de 1 s à 1000 s.

8. Ensemble selon l'une quelconque des revendications précédentes, le deuxième filtre absorbant (21) ayant un facteur de transmission supérieur ou égal à 10 % pour au moins une longueur d'onde inférieure à λ_{c}, et inférieur ou égal à 1% pour les longueurs d'onde du domaine visible supérieures ou égales à λ_{c}, avec λ_{c} inférieur à 850 nm.

9. Ensemble selon l'une quelconque des revendications précédentes, le spectre d'émission de la machine de traitement (1) étant sensiblement nul pour λ ≤ 580 nm, et supérieur ou égal à 100 W/cm² pour 650 ≤ λ ≤ 800 nm et notamment supérieur ou égal à 500 W/cm², pour au moins une longueur d'onde dans l'intervalle 600 nm à 850 nm.

10. Utilisation non-thérapeutique des lunettes ou du masque tels que définis dans l'une quelconque des revendications précédentes pour filtrer la lumière émise par la pièce à main.

## Patentansprüche

1. Anordnung, umfassend
- ein Pulslichtbehandlungsgerät (1), umfassend ein Handstück (4) mit einer Blitzleuchte (10) und einem ersten absorbierenden Filter (12) zum Filtern des von der Blitzleuchte emittierenden Lichts vor dessen Austreten aus dem Handstück,
- eine Brille (25) oder eine Schutzmaske des Bedieners mit einem zweiten absorbierenden Filter (21) zum Filtern des einfallenden Lichts,
wobei die Anordnung **dadurch gekennzeichnet ist, dass** die Gesamttransmission des sichtbaren und Infrarotlichts durch den ersten und den zweiten Filter im Emissionsbereich des Handstücks ausreichend gering ist, so dass der Bediener weder durch die Emission eines Blitzes visuell gestört noch durch das Infrarotlicht belastet wird, wobei die Gesamttransmission im Bereich 580 nm bis 1200 nm 10 % oder weniger beträgt, wobei der zweite absorbierende Filter im Emissionsbereich des Geräts von λ_{c} bis λ_{d} eine Transmission von 1 % oder weniger hat, wobei der Transmissionsfaktor der Brille (25) oder der Schutzmaske des Bedieners in einem Bereich zwischen 400 nm und λ_{c} 20 % oder mehr beträgt, wobei λ_{c} größer als 400 nm ist, wobei der absorbierende Filter der Brille oder der Maske bei λ < 400 nm einen Transmissionsfaktor von weniger als 1 % hat.

2. Anordnung nach Anspruch 1, wobei der zweite absorbierende Filter (21) außerhalb des Emissionsbereichs des Geräts eine Transparenz von mehr als 10 %, besser mehr als 95 % hat.

3. Anordnung nach Anspruch 1 oder 2, wobei die Gesamttransmission im Bereich λ_{c} bis λ_{d} weniger als 1 %, besser weniger als 0,1 %, noch besser weniger als 0,01 % beträgt, wobei λ_{c} kleiner als 850 nm und λ_{d} > λ_{c} ist.

4. Anordnung nach einem der Ansprüche 1 bis 3, wobei der absorbierende Filter (21) der Brille (25) oder der Maske blau ist.

5. Anordnung nach einem der Ansprüche 1 bis 4, wobei λ_{c} gleich 600 nm ist und λ_{d} zwischen 850 und 1200 nm liegt.

6. Anordnung nach einem der vorhergehenden Ansprüche, wobei das Handstück dem absorbierenden Filter (12) vorgelagert einen dichroitischen Filter (11) umfasst, der die Infrarotstrahlung filtert und die Gefahr einer Beschädigung des absorbierenden Filters verringert.

7. Anordnung nach einem der vorhergehenden Ansprüche, wobei das Gerät dazu eingerichtet ist, eine schnelle Folge von Blitzen zu emittieren, insbesondere von Blitzen, die mit einer Schussfrequenz von mindestens 1 Hz emittiert werden, insbesondere während einer Dauer von 1 s bis 1000 s.

8. Anordnung nach einem der vorhergehenden Ansprüche, wobei der zweite absorbierende Filter (21) einen Transmissionsfaktor von 10 % oder mehr bei mindestens einer Wellenlänge unterhalb von λ_{c} und von 1 % oder weniger bei Wellenlängen des sichtbaren Bereichs von λ_{c} oder darüber hat, wobei λ_{c} kleiner als 850 nm ist.

9. Anordnung nach einem der vorhergehenden Ansprüche, wobei das Emissionsspektrum des Behandlungsgeräts (1) bei λ ≤ 580 nm annähernd null ist und bei 650 ≤ λ ≤ 800 nm 100 W/cm² oder mehr beträgt und insbesondere bei mindestens einer Wellenlänge im Intervall 600 nm bis 850 nm 500 W/cm² oder mehr beträgt.

10. Nichttherapeutische Verwendung der Brille oder der Maske nach einem der vorhergehenden Ansprüche zum Filtern des vom Handstück emittierten Lichts.

## Claims

1. An assembly including:
- a pulsed light therapy machine (1) including a handset (4) including a flashlamp (10) and a first absorbing filter (12) for filtering the light emitted by the flashlamp before it exits the handset,
- a pair of glasses (25) or googles for protecting the operator, including a second absorbing filter (21) for filtering the incident light,
assembly **characterized in that** the overall transmittance of visible and infrared light through the first and second filters is sufficiently low in the range of emission of the HS that the operator is not visually discomforted by the emission of a flash, or harmed by the infrared light, the global transmission being less tha, or equal to 10% in the range 580 nm to 1200 nm, the second absorbing filter having a transmittance of 1% or less in the range λ_{c} to λ_{d} of emission of the machine, the transmission coefficient of the glasses (25) or of the googles being 20% or more in a range 400 to λ_{c}, with λ_{c} longer than 400 nm, the absorbing filter (21) of the glasses (25) or of the googles having a transmission coefficient lower than 1% both for λ < 400 nm.

2. The assembly as claimed in claim 1, the second absorbing filter (21) having a transparency higher than 10% and better still than 95% outside of the range of emission of the machine, said range preferably extending from 600 nm to 850 nm.

3. The assembly as claimed in claim 1 or 2, the overall transmittance in the range λ_{c} to λ_{d} being lower than 0.1% and better still lower than 0.01%, with λ_{c} shorter than 850 nm and λ_{d} > λ_{c}.

4. The assembly as claimed in any one of claims 1 to 3, the absorbing filter (21) of the glasses (25) or of the googles being of blue color.

5. The assembly as claimed in any one of claims 1 to 4, with λ_{c} equal to 600 nm and λ_{d} comprised between 850 and 1200 nm.

6. The assembly as claimed in any one of the preceding claims, the handset including upstream of the absorbing filter (12) a dichroic filter (11) that filter the infrared rays and reduce the degradation of the absorbent filter.

7. The assembly as claimed in any one of the preceding claims, the machine being arranged to emit a burst of flashes, in particular flashes emitted at a firing frequency of at least 1 Hz, in particular over a length of time that ranges from 1 s to 1000 s.

8. The assembly as claimed in any one of the preceding claims, the second absorbing filter (21) having a transmission coefficient of 10% or more at at least one wavelength shorter than λ_{c}, and of 1% or less at those wavelengths in the visible domain which are longer than or equal to λ_{c}, with λ_{c} shorter than 850 nm.

9. The assembly as claimed in any one of the preceding claims, the emission spectrum of the therapy machine (1) being substantially zero for λ ≤ 580 nm and better still λ ≤ 600 nm, and 100 W/cm² or more for 650 ≤ λ ≤ 800 nm and better still 640 ≤ λ ≤ 830 nm, and in particular 500 W/cm² or more at at least one wavelength in the interval 600 nm to 850 nm.

10. The non-therapeutic use of the glasses or googles such as defined in any one of the preceding claims to filter the light emitted by the handset.
